# EUROPEAN PATENT SPECIFICATION

(11) **EP 1 365 788 B1**
(45) Date of publication and mention of the grant of the patent: **09.05.2007**
(21) Application number: 01973674.3
(22) Date of filing: 31.08.2001
(51) Int. Cl.: A61K 38/36, A61P 25/00

(54) **THROMBOMODULIN ANALOGS FOR USE IN RECOVERY OF SPINAL CORD INJURY**
VERWENDUNG VON THROMBOMODULINANALOGA ZUR REGENERIERUNG VON RÜCKENMARKVERLETZUNGEN
ANALOGUES DE THROMBOMODULINE SERVANT A TRAITER UN TRAUMATISME MEDULLAIRE

(30) Priority: 31.08.2000 US 229714 P; 23.08.2001 US 938405
(43) Date of publication of application: 03.12.2003
(73) Proprietor: PAION Deutschland GmbH, 52062 Aachen (DE)
(72) Inventor: FESTOFF, Barry, W., Kansas City, MO 64112 (US); MORSER, Michael, John, San Francisco, CA 94114 (US)
(74) Representative: König, Gregor Sebastian
(86) International application number: PCT/US2001/041930
(87) International publication number: WO 2002/017953

(56) References cited:
- US-A- 5 256 770
- US-A- 5 466 668
- US-A- 5 863 760
- TAOKA Y ET AL: "Neuroprotection by recombinant thrombomodulin." THROMBOSIS AND HAEMOSTASIS. GERMANY MAR 2000, vol. 83, no. 3, March 2000 (2000-03), pages 462-468, XP008017328 ISSN: 0340-6245

## Description

This application claims the benefit of U.S. Provisional Application No. 60/229,714, filed August 31, 2000.

### FIELD OF THE INVENTION

The present invention relates to a method of using analogs of thrombomodulin for the manufacture of a medicament for the treatment of the neurologic trauma associated with spinal cord injury in mammals.

### BACKGROUND OF THE INVENTION

Thrombomodulin (TM) is a cell membrane glycoprotein. In humans, it is widely distributed on the endothelium of the vasculature and lymphatics. Its physiological importance has been extensively studied. (See, for example. Esmon et al., *J*. *Biol. Chem.* (1982), 257:859-864; Salem et al., *J*. *Biol. Chem.* (1983). 259:12246-12251).

Thrombomodulin functions as a receptor for thrombin, a central enzyme in the coagulation cascade. When free, thrombin promotes coagulation both directly by converting fibrinogen to fibrin, indirectly through activation of other proteins in the coagulation cascade (Factors V, VIII and XIII, for example), and through platelet activation. When bound to thrombomodulin, however, the thrombin-thrombomodulin complex is involved in activation of protein C to activated protein C, which then downregulates the coagulation cascade by proteolytically inactivating the essential cofactors Factor Va and Factor VIIIa (Esmon et al., *Ann. N. Y. Acad. Sci*. (1991), 614:30-43), resulting in increased anticoagulant activity. The thrombin-thrombomodulin complex also is involved in activation of thrombin-activatable fibrinolysis inhibitor (TAFI), which, when activated, leads to inhibition of fibrinolysis. Although earlier studies were negative, more recent studies have indicated that thrombomodulin is not only present in brain endothelial cells (Boffa, et al., *Nouv. Rev. Fr. Hematol*. (1991), 33:423-9; Wong, et al., *Brain Res.* (1991), 556:1-5; Wang, et al., *Arterioscler. Thromb. Vasc. Biol*. (1997), 17: 3139-46; Tran, et al., *Stroke* (1996), 27:2304-10; discussion 2310-1) but also is expressed on the surface of astrocytes, where it functions identically to its role in the vasculature, activating protein C by forming a complex with thrombin (Pindon, et al., *Glia* (1997), 19:259-68). Thrombomodulin is also upregulated in reactive astrocytes in the CNS, in response to mechanical injury (Pindon, et al., *J. Neurosci*. (2000), 20:2543-50). A recent report suggests that recombinant thrombomodulin block thrombin's activation of another receptor, the protease-activated receptor 1 (PAR-1) in cultured neuronal cells (Sarker, et al. *Thromb. Haemost*. (1999), 82: 1071-77).

Activated protein C has also been strongly implicated in the regulation of inflammatory responses involving various cytokines or activated leukocytes (Esmon et al., *Thromb. Haemost.* (1991), 66:160-165). Consistent with this hypothesis, studies have shown that activated protein C prevents pulmonary vascular injury in rats given endotoxin by inhibiting production of tumor necrosis factor (TNF-α), which potently activates neutrophils (Murakami et al., *Blood* (1996), 87:642-647; Murakami et al., *Am. J. Physiol*. (1996), 272:L197-2). Recombinant human soluble thrombomodulin also prevents-endotoxin-induced pulmonary vascular injury by inhibiting the activation of neutrophils through protein C activation (Uchiba et al., *Am. J. Physiol.* (1996), 271:L470-5; Uchiba et al., *Am. J. Physiol*. (1997), 273:L889-94).

Spinal cord injury (SCI) is a serious condition which produces life-long disabilities (Stover et al., *Paraplegia* (1987), 24:225-228). Only limited therapeutic measures are currently available for its treatment (Bracken et al., *New Engl. J. Med.* (1990), 322:1405-1411). In fact, the most commonly accepted acute intervention after SCI, other than surgery, is administration of the steroid, methylprednisolone (MP) (Hall, E. *D., Adv. Neurol*. (1993), 59: 241-8; Bracken, M. B., *J*. *Neurosurg.* (2000), 93:175-9; Bracken, M. B., *Cochrane Database Syst. Rev.* 2 (2000); Koszdin, et al., *Anesthesiology* (2000), 92:156-63). However, after 10 years of experience this treatment is still quite controversial and a recent meta analysis has suggested that treatment with MP may actually be contraindicated (Hurlbert, R. J., *J. Neurosurg*. (2000), 93:1-7; Pointillart, et al., *Spinal Cord* (2000), 38:71-6; Lankhorst, et al., *Brain Res.* (2000), 859:334-40).

The pathophysiology of SCI includes a primary mechanical injury and a delayed secondary neurological injury (Tator et al., *J. Neurosurg.* (1991), 75:15-26). Whereas the primary injury is determined by the circumstances of the trauma, the outcome of the secondary injury may be amenable to therapeutic modulation. Although the mechanisms involved in the secondary injury process are not-fully understood, inflammatory responses leading to endothelial damage may be involved (Demopoulos, et al., *Scan. Electron Microsc*. (1978), 2:677-680) and this is an area which can serve as a target for therapeutic intervention. Tumor necrosis factor (TNF-α) has recently been shown to play an important role in compression trauma-induced SCI in rats by activating neutrophils (Taoka, et al., *Neuroscience* (1997), 79:1177-182; Taoka et al., *J Neurotrauma* (2000), 17:219-29). It has also been reported that activated protein C reduces the severity of compression trauma-induced SCI by inhibiting TNF-α production (Taoka et al., *J*. *Neurosci*. (1998), 18:1392-1398). Studies have shown that recombinant soluble thrombomodulin prevented compression trauma-induced SCI in a rat SCI model by inhibiting leukocyte accumulation through reduction of TNF-α mRNA expression at the injured site (Taoka et al., *Thromb. Haemost*. (2000), 83:462-468). These observations suggest that thrombomodulin may also prevent contusion trauma-induced SCI through activation of protein C, with the resultant inhibition of TNF-α production. Contusion, or weight drop rat models have recently been validated for human SCI (Metz et al., *J Neurotrauma* (2000), 17: 1-17).

We have discovered that certain soluble thrombomodulin compositions are effective in reducing the neurologic damage following SCI in a rat model, and are therefore useful in the treatment of such neurologic damage in mammals. Soluble analogs of thrombomodulin that retain most, if not all, of the activities of the native protein have been produced. Furthermore, soluble analogs of thrombomodulin which are resistant to oxidation, resistant to proteolysis, or have in other ways been modified so as to possess a longer half-life within the circulation, have been developed and are described in U.S. Patent Nos. 5,256,770, 5,863,760 and 5,466,668. These compositions have previously been described as being useful as anti-thrombotic agents. However, there has not been any disclosure as to the usefulness of these compositions as therapeutic agents for the amelioration of neurologic damage following SCI.

### SUMMARY OF THE INVENTION

In accordance with the present invention, one aspect of this invention is directed to a method for manufacture of a medicament for treating the neurologic damage resulting from SCI which comprises a therapeutically effective amount of a soluble, recombinant thrombomodulin analog which is resistant to oxidation and wherein the methionine at position 388 has been replaced with a leucine, wherein the analog is numbered in accordance with native thrombomodulin (SEQ ID NO:2)

A further aspect of this invention utilizes thrombomodulin analogs which contain additional modifications to provide resistance to protease cleavage and/or show an altered pattern of glycosylation.

A further aspect of this invention utilizes a thrombomodulin analog, known as Solulin^{™}, which contains modifications to the sequence of native thrombomodulin (SEQ ID NO: 2) at the following positions: removal of amino acids 1-3, M388L, R456G, H457Q, S474A, and termination at P490.

### Brief Description of the Drawings

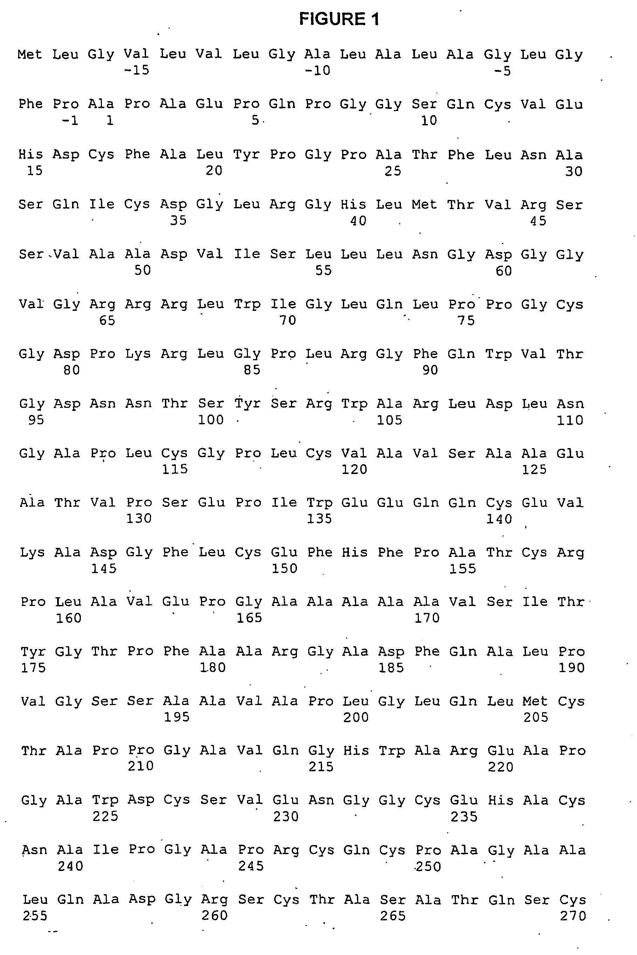

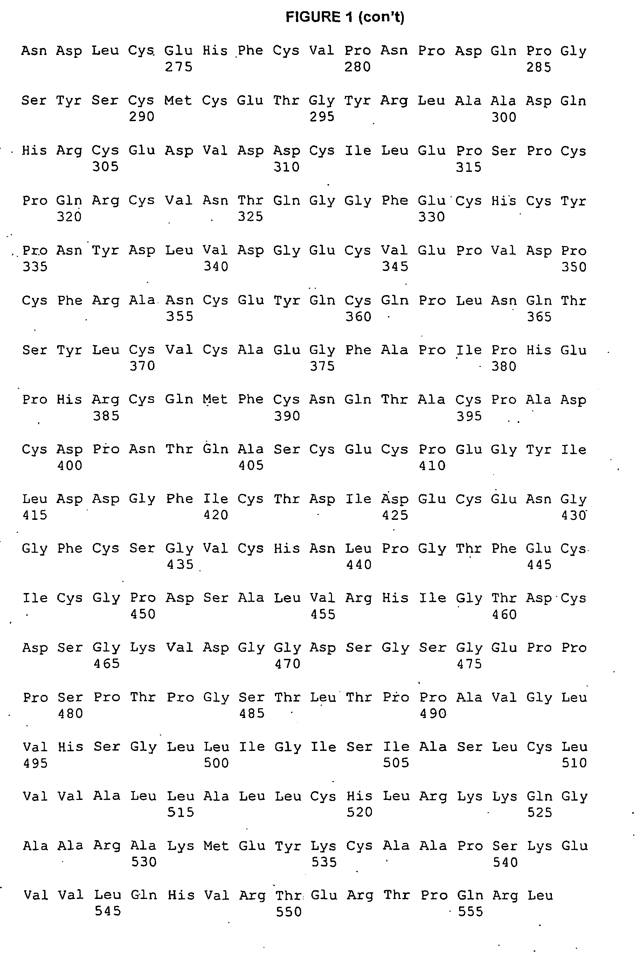

FIG 1 shows the amino acid sequence of native thrombomodulin (SEQ ID NO: 2), using the numbering system of Suzuki et al. (1987) *Embo J* **6**: 1891-1897.

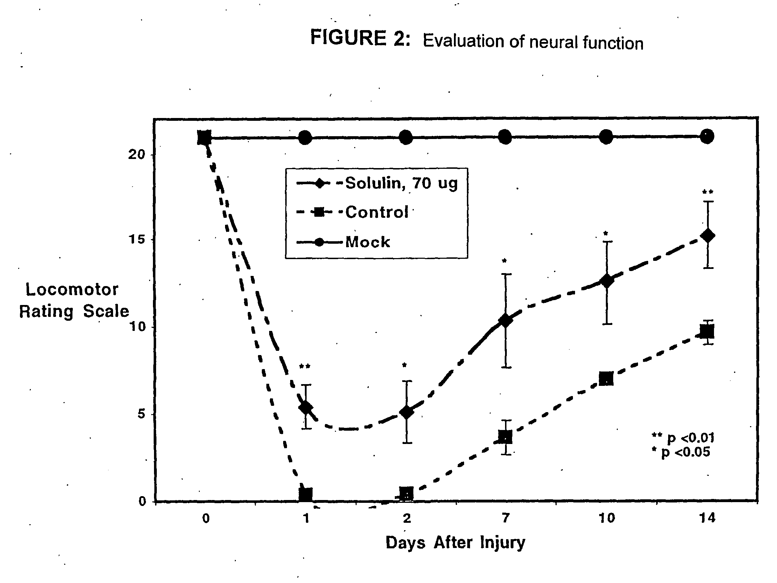

FIG 2 shows results of evaluation of neural function by the open-field locomotor rating scale (LRS) of Basso, Beatty and Bresnahan, the so-called "BBB" scale, in rats following controlled contusion spinal cord injury. Injured rats are either treated with vehicle (saline) or with Solulin^{™} administered intraperitoneally (i.p.) post-injury as described in Example 2.

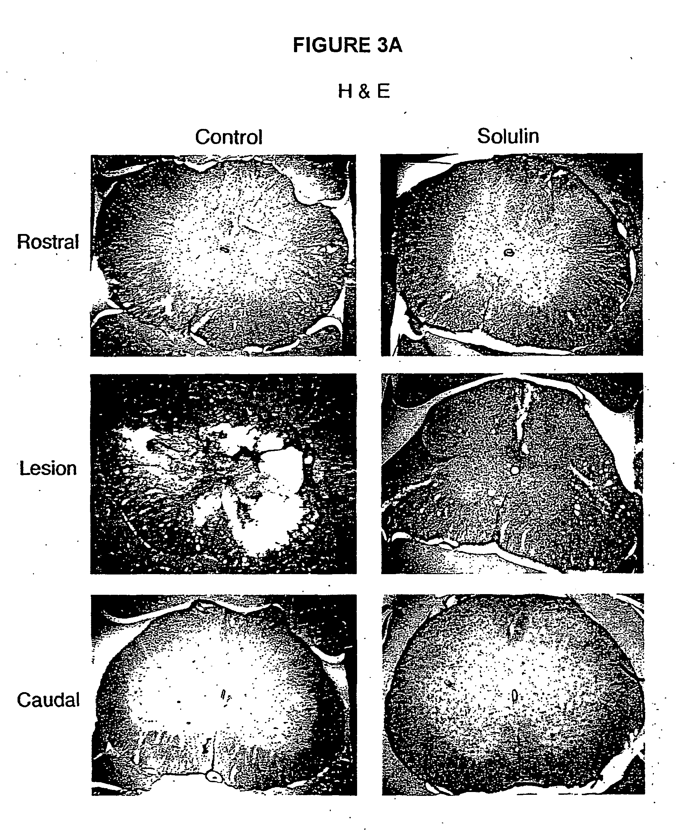

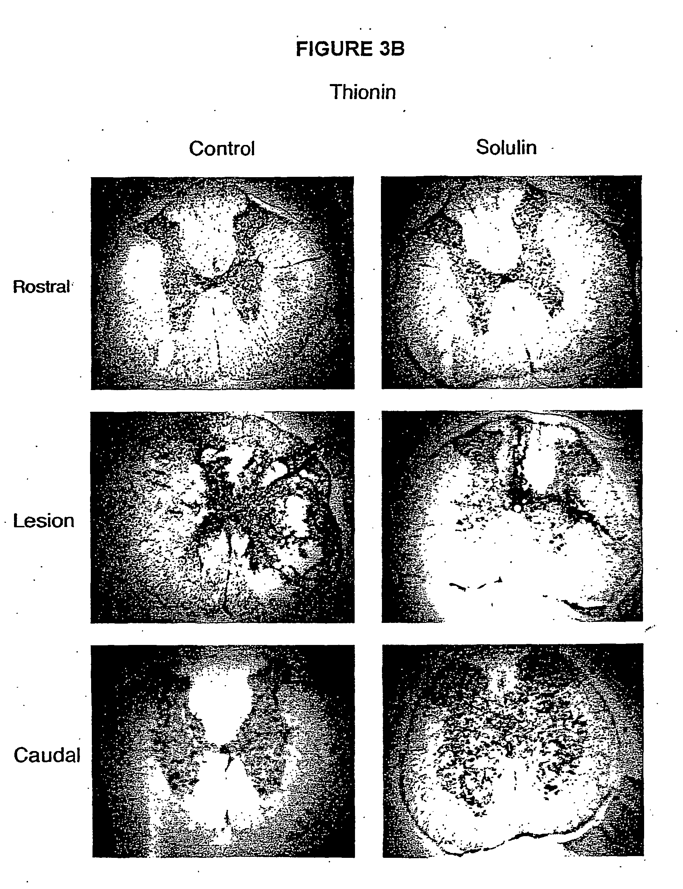

FIG 3 (A and B) illustrates representative histologic specimens from rats with spinal cord injury following treatment with saline (Control) or with Solulin^{™} given i.p at 1h following moderate contusion SCI (25 gm.cm force). Specimens from above, at, and below the injury are shown. In Figure 3A, the specimens were stained with hematoxylin and eosin (H&E); in Figure 3B, the specimens were stained with thionin.

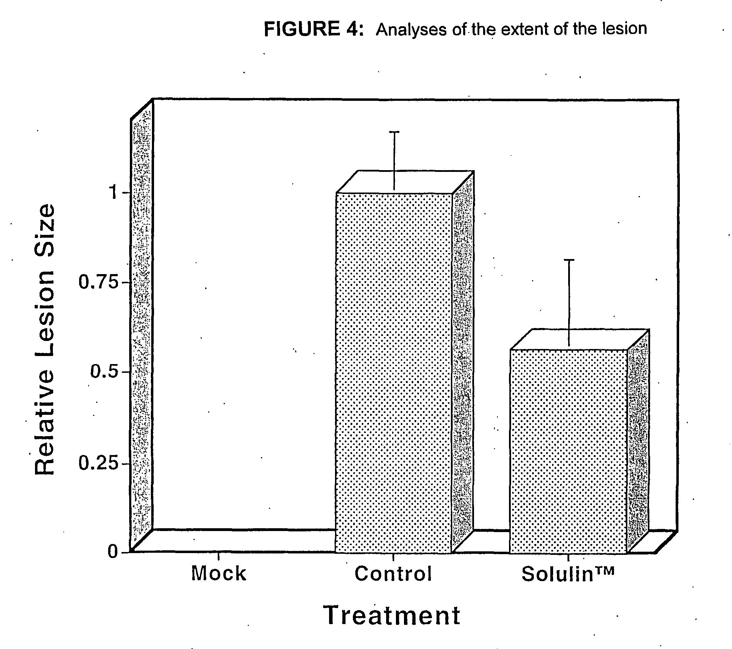

FIG 4 shows analyses of the extent of lesion volume as determined by histological examination in saline (control) versus Solulin^{™} treated rats, at, above and below the lesion epicenter. A statistically-significant reduction of about 40% (p <0.05) in lesion volume was found with Solulin^{™} treatment as compared to saline treated controls with an unpaired t-test.

### DETAILED DESCRIPTION OF THE INVENTION

### Definitions

As used in the specification and claims, unless specified to the contrary, the following terms have the meaning indicated:

The term "residue" refers to an amino acid that is incorporated into a peptide. The amino acid may be a naturally occurring amino acid and, unless otherwise limited, may encompass known analogs of natural amino acids that can function in a similar manner as naturally occurring amino acids. For purposes of this disclosure, amino acid residues are designated herein by their accepted three-letter or one-letter abbreviation, or by the notation "AA", which signifies the presence of an amino acid residue. The amino acids referred to herein are described by shorthand designations as follows:

**Table 1: Amino Acid Nomenclature**

| Name | 3-letter | 1 letter |
|---|---|---|
| Alanine | Ala | A |
| Arginine | Arg | R |
| Asparagine | Asn | N |
| Aspartic Acid | Asp | D |
| Cysteine | Cys | C |
| Glutamic Acid | Glu | E |
| Glutamine | Gln | Q |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

When describing an amino acid substitution, for purposes of this disclosure, the substitution is described by providing the amino acid present in native thrombomodulin (SEQ ID NO: 2) (TM), the location of the amino acid within the thrombomodulin sequence (using the numbering system of Suzuki et al, *Embo Journal* (1987), 6:1891-1897), followed by the amino acid which has been substituted for the original: i.e. M388L refers to substitution of methionine at position 388 with leucine).

"Native thrombomodulin" refers to the full length protein as it occurs in nature (FIG 5: SEQ ID NO: 2). Native thrombomodulin is known to contain naturally occurring polymorphisms at certain residues. For example, at position 455, there is a naturally occurring variation in the amino acid found at this position, with an alanine present 82% of the time and a valine present 18% of the time (Van der Velden et al. (1991) *Throm. Haemeostasis* 65:511-513.) For purposes of this invention, the native thrombomodulin sequence shown (FIG 5; SEQ ID NO: 2) is one which contains valine at position 455, as described by Suzuki et al. (1987) *EMBO J* 6:1891-1897. However, all naturally occurring polymorphisms are included within the scope of the claimed analogs. When biological activities are described with reference to the native TM, the term embraces a detergent solubilized aqueous form. Often, in the context of comparison to an activity, a transfected soluble polypeptide may exhibit substantially identical properties.

"Thrombomodulin analogs" are peptides which substantially retain the biological activity of native TM, as discussed above, and which have a molecular structure different from that of a native version TM. For example, the term refers to proteins having an amino acid sequence identical or homologous with that of native thrombomodulin (SEQ ID NO: 2), to insoluble and soluble thrombomodulin peptides or fragments, and to oxidation resistant TM species, all having thrombomodulin-like activity. These compounds also include derivatives and molecules comprising amino acid changes which do not significantly decrease the protein C activation cofactor properties of the protein when compared with native TM.

The term "TM mutant" refers to a TM analog containing the designated substitution (as described above) or other indicated modification.

The terms "peptides" and "polypeptides" refer to chains of amino acids whose α carbons are linked through peptide bonds formed by a condensation reaction between the α carbon carboxyl group of one amino acid and the amino group of another amino acid. The terminal amino acid at one end of the chain (amino terminus) therefore has a free amino group, while the terminal amino acid at the other end of the chain (carboxy terminus) has a free carboxyl group.

The term "domain" refers to a discrete amino acid sequence that can be associated with a particular function or characteristic. Typically, a domain exhibits a characteristic tertiary structural unit. The full-length thrombomodulin gene encodes a precursor peptide containing the following domains:

**Table 2: TM Domains**

| Approximate Amino Acid Position | Domain |
|---|---|
| (-18)-(-1) | Signal sequence |
| 1-226 | N-terminal domain (lectin domain; L) |
| 227-462 | 6 EGF-like domains (E) |
| 463-497 | O-linked Glycosylation (D) |
| 498-521 | Transmembrane |
| 522-557 | Cytoplasmic domain |

See Yost et al., *Cell*, (1983), 34:759-766 and Wen et al., *Biochemistry* (1987), 26:4350-4357.

A "protease site" refers to an amino acid or series of amino acids in a TM. polypeptide which define a recognition, binding, cleavage, or other site susceptible to the activity of a protease, for example, when one or more amino acid residues encompassed by this site are substituted by another amino acid residue(s) or are deleted, the protease is no longer able to cleave the TM at that site. This term also encompasses regions of the TM molecule which are inherently susceptible to proteases, e.g., by being conformationally exposed and available to a protease activity.

A "protease cleavage site" refers to the precise location at which a protease cleaves the TM polypeptide analog.

A "single N-terminus" and "single C-terminus" have their literal meanings which functionally refer to the property of the composition, e.g., wherein, upon conventional sequential amino acid sequence analysis, each degradation cycle results in the removal of an amino acid residue which is essentially devoid of a different amino acid residue. Thus, after several cycles, e.g., 10 cycles, of stepwise removal of the N-terminal amino acids, essentially only one amino acid is recovered at each cycle. In particular, no more heterogeneity in sequence is detected than would be statistically expected from a completely pure single-chain polypeptide according to the analytic procedure used.

"Substantially retains the biological activity of native thrombomodulin (SEQ ID NO: 2)" and similar terms, as used herein, means that the thrombomodulin shares biological activities with a native membrane bound TM molecule. Generally, the activity in units per milligram of protein is at least about 50%, ordinarily 75%, typically 85%, more typically 95%, preferably 100% and more preferably over 100% of the activity of native thrombomodulin (SEQ ID NO: 2). This biological activity can be that of thrombin-mediated activation of protein C (APC), of activated partial thromboplastin clotting time (APTT), of thrombin clotting time (TCT), or of any of TM's biological, preferably therapeutic, activities. The native standard of comparison is a full-length membrane bound version of TM, but in many cases, a soluble TM comprising the lectin/EGF/O-linked domain (TM.sub.LEO) can be used as a more convenient standard.

"Glycosylation sites" refer to amino acid residues which are recognized by a eukaryotic cell as locations for the attachment of sugar residues. The amino acids where sugars are attached are typically Asn (for N-linked sugars), threonine or serine (for O-linked sugars) residues. The specific site of attachment is typically signaled by a sequence of amino acids, e.g., Asn-X-(Thr or Ser) for most N-linked attachment and (Thr or Ser)-X-X-Pro for most O-linked attachment, where X is any amino acid. The recognition sequence for glycosaminoglycans (a specific type of sulphated sugar) is generally Ser-Gly-X-Gly, but can also be X-Ser-Gly-X-Val. The terms N-linked and O-linked refer to the chemical group that serves as the attachment site between the sugar moiety and the amido acid residue. N-linked sugars are attached through an amino group; O-linked sugars are attached through an hydroxyl group.

"In vivo circulating half-life" refers to the average time it takes an administered plasma activity in a mammal to decrease by one half.

A "soluble TM analog" is a TM analog which is soluble in an aqueous solution, and typically can be secreted by a cell. For pharmacological administration, the soluble TM analog or an insoluble analog may optionally be combined with phospholipid vesicles, detergents, or other similar compounds well known to those skilled in the art of pharmacological formulation. The preferred TM analogs of the present invention are soluble in the blood stream, making the analogs useful in various anticoagulant and other therapies. The modifications which make TM soluble typically do not significantly affect many activities relative to native thrombomodulin (SEQ ID NO: 2), e.g., affinity for thrombin or activity in protein C activation.

"O-linked glycosylation domain" refers to the sequence of amino acids numbered from 463 through 497 of the native thrombomodulin sequence (SEQ ID NO: 2), as depicted in Table 2 (see page 5).

"Oxidation resistant analogs" refers to analogs of thrombomodulin which are able to maintain a substantial amount of biological activity after exposure to an oxidizing agent such as oxygen radicals, Chloramine T, hydrogen peroxide, or activated neutrophils.

"Solulin^{™} refers to a thrombomodulin analog described in U.S. Patent No. 5,256,770, in which the following modifications to the sequence of native thrombomodulin (SEQ ID NO: 2) have been made: removal of amino acids 1-3, M388L, R456G, H457Q, S474A, and termination at P490.

"Pharmaceutical excipients" refers to non-toxic, medically-acceptable materials which are used to complete a medical therapeutic. These materials can be inert, such as water and salt, or biologically active, such as an antibiotic or analgesic.

"Reduced ability" refers to a statistically meaningful lowering of a biological property. The property is unlimited and the measurement or quantification of the property is by standard means.

"Sugar residues" refers to hexose and pentose carbohydrates including glucosamines and other carbohydrate derivatives and moieties which are covalently linked to a protein.

"Sulfate substituents" are sulfur-containing substituents on pentose or hexose sugars.

"Thrombin-mediated, conversion of fibrinogen to fibrin" refers to the enzymatic activity by which thrombin cleaves the precursor protein fibrinogen to make fibrin monomer, which subsequently polymerizes to form a blood clot.

"Thrombotic disease" refers to a pathogenic condition in a mammal characterized by the formation of one or more thrombi that are or can be detrimental to the health of the mammal.

"SCI" as used herein refers to traumatic injuries sustained to the spinal cord and the area around it. This includes contusion and/or compression injuries, as well as transection injury. The model used in the studies supporting the utility of this invention is contusion, which most closely approximates the types of SCI suffered by humans in motor vehicle accidents and/or sports-related injuries. All SCI is characterized by sudden loss of complete or partial motor function and the extent of this loss depends on the location within the spine of the injuries. Higher (cervical) injuries can result in total loss of motor function or quadraplegia and loss of respiratory control, and sometimes cardiovascular collapse. Lower lesions can result in paraplegia but without arm involvement or respiratory dysfunction.

"Mammal" includes humans and domesticated animals, such as cats, dogs, swine, cattle, sheep, goats, horses, rabbits, and the like.

"Therapeutically effective amount" refers to that amount of thrombomodulin analog, which, when administered to a mammal in need thereof, preferably a human, is sufficient to effect treatment, as defined below, for neurologic damage resulting from SCI. The amount of thrombomodulin analog which constitutes a "therapeutically effective amount" will vary depending on the thrombomodulin analog, the severity of SCI, and the age of the mammal to be treated, but can be determined routinely by one of ordinary skill in the art having regard to his own knowledge and to this disclosure.

"Treating" or "treatment" as used herein covers amelioration of the neurologic damage associated with SCI in a mammal, preferably a human, which damage is associated with loss of motor and/or respiratory function, and includes treatment which results in improved recovery of neurologic function.

### Utility of the Invention

This invention is directed to a method for the manufacture of a medicament for the treatment in mammals of the neurologic trauma associated with spinal cord injury (SCI). As discussed above, activated protein C inhibits the production of TNF-α, a molecule that has been shown to play an important role in compression trauma-induced SCI, and it is known that thrombomodulin, in complex with thrombin, produces activated protein C. The instant invention provides a medicament for treating mammals having SCI, comprising a thrombomodulin analogs which possess the same activity as native thrombomodulin (SEQ ID NO: 2), but which exhibit properties which make the analogs better therapeutic agents.

To demonstrate the utility of the thrombomodulin analogs of the invention as therapeutic agents for treatment of the neurologic trauma associated with SCI, thrombomodulin analogs were evaluated for their ability (1) to improve locomotor rating scale (LRS) scores, which is a method of evaluating spinal cord function, and (2) to improve spinal cord histology, which provides a picture of spinal cord healing, in rats following SCI (see Figures 2-4).

As an additional indication of the utility of parenteral administration of the thrombomodulin analog (Solulin^{™}) in this context, it has been ascertained that intraperitoneal (i.p) administration of Solulin^{™} has significant effects on plasma clotting functions.

Studies indicate that early therapeutic intervention (1-3 hrs post injury) is preferred.

### Animal Models for SCI

Several experimental systems have been used to investigate the pathophysiology of SCI and to test the effects of neuroprotective agents in the laboratory (Amar and Levy, *Neurosurgery* (1999), 44:1027-1040). Current experimental paradigms involve neuronal cell cultures or anatomically intact segments of spinal cord subjected to various mechanical or ischemic insults, such as weight drop, focal or circumferential extradural balloon compression, clip pressure, photochemical or thermal injury, distractional forces, or piston trauma.

A more preferred method which more closely approximates human SCI (Metz, et al., *J*. *Neurotrauma* (2000), 17:1-17) is the infliction of spinal cord contusion according to the Multi-Center Animal Spinal Cord Injury Study (MASCIS) protocol using the controlled contusion weight drop method (Gruner, J. A, *J. Neurotrauma* (1992), 9:123-6; Basso, et al., *J. Neurotrauma* (1996), 13:343-59). The resultant injury can be assessed by histological examination (e.g. light or electron microscopy and special staining and tracing methods) (Gruner, J. A., Ibid.), electrophysiological outcome measures (e.g., evoked potentials) (Metz, et al., *J. Neurotrauma* (2000), 17:1-17), or behavioral assessments (e.g., open field locomotion or postural stability on an inclined plane) (Basso, et al., *J. Neurotrauma* (1996), 13:343-59).

### Laboratory Assays for Measuring TM Activity

A number of laboratory assays for measuring TM activity are available. Protein C cofactor activity can be measured in the assay described by Salem, et al., *J. Biol. Chem*. (1984), 259(19):12246-12251 and Galvin, et al., *J. Biol. Chem.* (1987), 262(5):2199-2205. In brief, this assay consists of two steps. The first is the incubation of the test TM analog with thrombin and protein C under defined conditions. In the second step, the thrombin is inactivated with hirudin or antithrombin III and heparin, and the activity of the newly activated protein C is determined by the use of a chromogenic substrate, whereby the chromophore is released by the proteolytic activity of activated protein C. This assay is carried out with purified reagents.

Alternatively the effect of a TM analog can be measured using plasma in clotting time assays such as the activated partial thromboplastin time (aPTT), thrombin clotting time (TCT), and/or prothrombin time (PT). The aPTT assay is dependent on both the activating of protein C, as well as the direct inhibition of thrombin, while the TCT and PT assays are dependent only on the inhibition of thrombin. Prolongation of the clotting time in any one of these assays demonstrates that the molecule can inhibit coagulation in plasma. Assays can be run on an automatic coagulation timer according to the manufacturer's specifications; Medical Laboratory Automation Inc. distributed by American Scientific Products, McGaw Park, III. (See also Salem et al., *J*. *Biol. Chem*. (1984), 259:12246-12251).

TAFI activation can be measured as described by Wang et al. (*J. Biol. Chem*. (2000), 275:22942-22947), utilizing the fact that activated TAFI is a carboxypeptidase. In this assay, extracts containing the thrombomodulin analog in question are incubated with thrombin, and the mixture then incubated with purified TAFI. The amount of activated TAFI produced is determined by the use of a chromogenic substrate, whereby the chromophore is released by the proteolytic activity of activated TAFI. Alternatively, TAFI activation can be assayed by a plasma clot lysis assay either in a defined system using purified proteins or in a plasma milieu (Nagashima, et al., *Throm. Research* (2000), 98:333-342).

The assays described above are used to identify soluble TM analogs that are able to bind thrombin and to assess the ability of the thrombin-thrombomodulin complex formed with these analogs to activate protein C, both in purified systems and in a plasma milieu. Further assays can be used to evaluate other activities of native thrombomodulin (SEQ ID NO: 2) such as inhibition of thrombin catalyzed formation of fibrin from fibrinogen (Jakubowski, et al., *J. Biol. Chem.* (1986), 261(8):3876-3882), inhibition of thrombin activation of Factor V (Esmon, et al., *J. Biol. Chem*. (1982), 257:7944-7947), accelerated inhibition of thrombin by antithrombin III and heparin cofactor II (Esmon, et al., *J. Biol. Chem*. (1983) 258:12238-12242), inhibition of thrombin activation of Factor XIII (Polgar, et al., *Thromb. Haemostas*. (1987), 56:140), inhibition of thrombin mediated inactivation of protein S (Thompson and Salem, *J. Clin. Inv*. (1986), 78(1):13-17) and inhibition of thrombin mediated platelet activation and aggregation (Esmon, et al., *J. Biol. Chem*. (1983), 258:12238-12242).

### Modifications to thrombomodulin

Modifications to the native thrombomodulin (SEQ ID NO: 2) molecule are useful to increase the therapeutic effectiveness of the thrombomodulin analogs of the present invention.

Particularly preferred TM analog compositions are those that have one or more of the following characteristics:
(i) they are oxidation resistant,
(ii) they exhibit protease resistance,
(iii) they have homogeneous N- or C-termini,
(iv) they have been post-translationally modified, e.g., by glycosylation of at least some of the glycosylation sites of native thrombomodulin (SEQ ID NO: 2).
(v) they have linear double-reciprocal thrombin binding properties,
(vi) they are soluble in aqueous solution in relatively low amounts ot detergents and typically lack a transmembrane sequence.

These modifications have been described in U.S. Patent Nos. 5,256,770, 5,863.760, and 5,466,668.

In particular, preferred modifications to the TM molecule which relate to these characteristics include removal of amino acids 1-13, termination at P490, and the following substitutions: M388L (for oxidation resistance), R456G and H457Q (both for proteolysis resistance) and S474A (blocks glycosaminoglycan addition and slows clearance).

Most preferred is a molecule comprising all of these modifications, which is referred to as Solulin^{™}.

### Preparation of the TM analogs of this Invention

Preparation of the TM analogs used in this invention is disclosed in U. S. Pat. Nos. 5,256,770, 5,863,760, and 5,466,668.

### General Administration of the medicament according to the invention comprising

The medicament Thrombomdolin analogs comprising input form Administration of the medicament comprising thrombomodulin analogs of the invention in pure form or in an appropriate pharmaceutical composition, can be carried out via any of the accepted modes of administration or agents for serving similar utilities. Thus, administration can be, for example, orally, nasally, parenterally, topically, transdermally, or rectally, in the form of solid, semi-solid, lyophilized powder, or liquid dosage forms, such as for example, tablets, suppositories, pills, soft elastic and hard gelatin capsules, powders, solutions, suspensions, or aerosols, or the like, preferably in unit dosage forms suitable for simple administration of precise dosages. The compositions will include a conventional pharmaceutical carrier or excipient and a compound of the invention as the/an active agent, and, in addition, may include other medicinal agents, pharmaceutical agents, carriers, adjuvants, etc.

Generally, depending on the intended mode of administration, the pharmaceutically acceptable compositions will contain about 1% to about 99% by weight of a compound(s) of the invention, or a pharmaceutically acceptable salt thereof, and 99% to 1% by weight of a suitable pharmaceutical excipient. Preferably, the composition will be about 5% to 75% by weight of a compound(s) of the invention, or a pharmaceutically acceptable salt thereof, with the rest being suitable pharmaceutical excipients.

The compounds of the invention, or their pharmaceutically acceptable salts, may also be formulated into a suppository using, for example, about 0.5% to about 50% active ingredient disposed in a carrier that slowly dissolves within the body, *e*.*g*., polyoxyethylene glycols and polyethylene glycols (PEG), e.g., PEG 1000 (96%) and PEG 4000 (4%).

The preferred route of administration is parenterally, for example, by injection. Injection can be subcutaneous, intravenous or intramuscular. These analogs are administered in pharmaceutically effective amounts and often as pharmaceutically acceptable salts, such as acid addition salts. Such salts can include, e.g., hydrochloride, hydrobromide, phosphate, sulphate, acetate, benzoate, malate, citrate, glycine, glutamate, and aspartate, among others. See Goodman & Gilman's, *The Pharmacological Basis of Therapeutics*, 8^{th} ed., Pergamon Press, 1985, which is incorporated herein by reference. Liquid pharmaceutically administrable compositions can, for example, be prepared by dissolving, dispersing, *etc*., a compound(s) of the invention (about 0.5% to about 20%), or a pharmaceutically acceptable salt thereof, and optional pharmaceutical adjuvants in a carrier, such as, for example, water, saline, aqueous dextrose, glycerol, ethanol and the like, to thereby form a solution or suspension.

If desired, a pharmaceutical composition of the invention may also contain minor amounts of auxiliary substances such as wetting or emulsifying agents, pH buffering agents, antioxidants, and the like, such as, for example, citric acid, sorbitan monolaurate, triethanolamine oleate, butylated hydroxytoluene, *etc*.

Actual methods of preparing such dosage forms are known, or will be apparent, to those skilled in this art; for example, see *Remington's Pharmaceutical Sciences,* 18th Ed., (Mack Publishing Company, Easton, Pennsylvania, 1990), which is incorporated herein by reference. The composition to be administered will, in any event, contain a therapeutically effective amount of a compound of the invention, or a pharmaceutically acceptable salt thereof, for treatment of a disease-state alleviated by the reduction of plasma levels of Lp(a) or by the inhibition of the generation of apo(a) in accordance with the teachings of this invention.

The medicament manufactured according to the invention comprises a therapeutically effective amount of thrombomodulin which will vary depending upon a variety of factors including the activity of the specific compound employed, the metabolic stability and length of action of the compound, the age, body weight, general health, sex, diet, mode and time of administration, rate of excretion, drug combination, the severity of the particular disease-states, and the host undergoing therapy. Generally, these medicaments can be administered to mammals for veterinary use, such as with domestic animals, and for clinical use in humans in a manner similar to other therapeutic agents, that is, in a physiologically acceptable carrier. In general, the administration dosage for the TM analog will range from about at least 0.0002, more usually 0.02, and less than 5000, usually less than 2000, more usually less than 500 µg/kg, usually 0.02 to 2000 µg/kg and more usually 0.02 to 500 µg/kg of the host body weight. These dosages can be administered by constant infusion over an extended period of time, until a desired circulating level has been attained, or preferably as a bolus injection. Optimal dosages for a particular patient can routinely be determined by one of ordinary skill in the art.

Without further elaboration, it is believed that one skilled in the art can, using the preceding description, utilize the present invention to its fullest extent. The following preferred specific embodiments are, therefore, to be construed as merely illustrative and not limitative of the remainder of the disclosure in any way whatsoever.

In the foregoing and in the following examples, all temperatures are set forth uncorrected in degrees Celsius; and, unless otherwise indicated, all parts and percentages are by weight.

While the present invention has been described with reference to the specific embodiments thereof, it should be understood by those skilled in the art that various changes may be made and equivalents may be substituted without departing from the true spirit and scope of the invention. In addition, many modifications may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### EXAMPLES

### Example 1: Rat Model for SCI

Animals: Adult female Sprague-Dawley rates (Charles River, NY) weighing 270-325 g are housed at 12-hour light-dark cycle and fed rodent chow *ad libitum* and were given tap water to drink. All animal experiments are carried out in the Animal Care Facility, under NIH Guidelines for animal studies. Only animals in good health are used. One week before the surgical procedures, animals are handled on a daily basis to adapt them for open field locomotor rating scale measurements.

Neurosurgical procedures: Animals are anesthetized with an intraperitoneal injection of ketamine (80 mg/kg) and xylazine (5 mg/kg). The wound site is prepared for contusion SCI using the NYU impactor by shaving and sterilizing the area of the incision over the dorsal lower thoracic area according to the MASCIS protocol as described (Gruner, J. A. *J. Neurotrauma* (1992), 9:123-6: Basso, et al., *J. Neurotrauma* (1996), 13: 343-59).

Prior to injury, blood pressure is monitored, arterial blood is collected for gas measurements, and rectal temperature is recorded.

### Example 2: Contusion and Post-Contusion Procedures

Contusion: Spinal cord contusion is performed using the controlled contusion weight-drop method with an NYU impactor, using the protocol described above (Gruner, J.A. *J. Neurotrauma* (1992), 9:123-126; Yong, et al., *J. Neurotrauma* (1998) 15:459-472).

Post-injury Procedures: During the 48 hours after injury, treatment is delivered, data collected, and blood/urine samples collected. About a third of the rats are euthanized at 48 hours after injury for determination of acute lesion volume. The remainder are maintained for from 14 to 28 days after injury to allow motor function determinations (LRS/BBB) to be made.

Solulin^{™} Treatment One hour after injury, a single injection of 70 µg Solulin^{™} dissolved in 200 µl normal saline is given.i.p. to a group of three rats. Vehicle control animals, which had undergone complete injury as per the treatment group, received only saline. In some experiments, a second dose of 70 µg Solulin^{™} dissolved in 200 µl normal saline is given i.p. 24 h after impact Control (sham-injured) animals underwent all surgical manipulations, including laminectomy, with the exception of weight drop injury.

aPTT measurement Blood for plasma activated partial thromboplastin time (aPTT) levels is collected at 3, 6, 12, 24 and 72 hrs after SCI. The blood is withdrawn from the tail vein using a 1 ml tuberculin syringe, withdrawing approximately 1 ml of blood into premeasured acid citrated tubes. The blood is immediately centrifuged, plasma removed, and frozen at -70° until aPTT levels can be measured (Salem et al., *J. Biol. Chem*. (1984), 259L12246-12251). aPTT level measurements demonstrate that thrombomodulin activity is detectable for at least 24 hours post injection.

At least three separate experiments with three rats per group of Solulin^{™} -treated animals and vehicle control were utilized.

Evaluation of neurologic damage: Open field locomotor rating scale (LRS/BBB) measurements (Basso, et al., *J. Neurotrauma* (1996) 13: 343-59; Basso et al., *Exp. Neurol*. (1996) 139:244-256) were performed by 3 separate blinded observers over a 24 day period after SCI. Results are recorded and entered into a software program developed for the LRS and analyzed.

### Example 3: Histological Examination of Spinal Cord Tissue

Fixed spinal cord samples are embedded and sectioned both longitudinally and horizontally to evaluate and measure the area of the lesion site at injury and at adjacent segments with hematoxylin and eosin (H & E), thionin and other stains. See Figure 3. (Bethea, et al., *J*. *Neurotrauma* (1999), 16:851-63).

Figure 4 shows an analysis of the lesion volume and indicates that a statistically significant reduction in lesion volume was found with Solulin^{™} treatment.

## Claims

1. The use of a therapeutically effective amount of a soluble, recombinant thrombomodulin analog which is resistant to oxidation and wherein the methionine at position 388 has been replaced with a leucine, wherein the analog is numbered in accordance with native thrombomodulin SEQ ID NO: 2 for the manufacture of a medicament for the treatment of Spinal Cord Injuries.

2. The use of claim 1, wherein the thrombomodulin analog is modified in the sugar residues of the O-linked glycosylation domain of native thrombomodulin SEQ ID NO: 2.

3. The use of claim 1 or 2, wherein the thrombomodulin analog is modified such that the O-linked glycosylation domain has no chondroitin sulfate.

4. The use of one of the preceding claims, wherein the analog has been rendered resistant to protease cleavage.

5. The use of one of the preceding claims, wherein the thrombomodulin analog (Solulin^{™}) has the amino acid sequence of native thrombomodulin (SEQ ID NO: 2) modified at the following positions:
removal of amino acids 1-3
M388L
R456G
H457Q
S474A, and
terminating at P490.

6. The use of one of the preceding claims wherein the mammal is a human.

## Patentansprüche

1. Die Verwendung einer therapeutisch effektiven Menge eines löslichen, rekombinanten Thrombomodulin-Analogons, das resistent gegenüber Oxidation ist, wobei das Methionin an der Position 388 durch ein Leukin ersetzt wurde, wobei das Analogon gezählt wird in Übereinstimmung mit der nativen Thrombomodulin-Sequenz ID NO. 2, zur Herstellung eines Medikaments für die Behandlung von Rückenmarksverletzungen.

2. Die Verwendung nach Anspruch 1, wobei das Thrombomodulin-Analogon in den Zuckerresten der O-verbundenen Glukosylierungsdomain der nativen Thrombomodulin-Sequenz ID NO. 2 modifiziert ist.

3. Die Verwendung nach Anspruch 1 oder 2, wobei das Thrombomodulin-Analogon derart modifiziert ist, dass die O-verbundene Glukosylierungsdomain kein Chondroitin-Sulfat aufweist.

4. Die Verwendung nach einem der vorangehenden Ansprüche, wobei das Analogon resistent gemacht wurde gegen eine Proteasespaltung.

5. Die Verwendung nach einem der vorherigen Ansprüche, wobei das Thrombomodulin-Analogon (Solulin^{™}) die Aminosäuresequenz des nativen Thrombomodulins (SEQ ID NO. 2) aufweist, die an folgenden Positionen modifiziert wurde:
Entfernung der Aminosäurereste 1 bis 3
M388L
R456G
H457Q
S474A und
bei P490 endet.

6. Die Verwendung nach einem der vorherigen Ansprüche, wobei der Säuger ein Mensch ist.

## Revendications

1. Utilisation d'une quantité thérapeutiquement efficace d'un analogue de thrombomoduline recombinant soluble qui est résistant à l'oxydation et dans lequel la méthionine à la position 388 a été remplacée par une leucine, dans laquelle l'analogue est numéroté conformément à la thrombomoduline native de la SEQ ID N° : 2, pour la fabrication d'un médicament destiné au traitement de lésions de la moelle épinière.

2. Utilisation selon la revendication 1, dans laquelle l'analogue de thrombomoduline est modifié dans les résidus de sucre du domaine de glycosylation lié à O de la thrombomoduline native de la SEQ ID N° : 2.

3. Utilisation selon la revendication 1 ou la revendication 2, dans laquelle l'analogue de thrombomoduline est modifié de sorte que le domaine de glycosylation lié à O ne possède pas de sulfate de chondroïtine.

4. Utilisation selon l'une quelconque des revendications précédentes, dans laquelle l'analogue a été rendu résistant au clivage par protéase.

5. Utilisation selon l'une quelconque des revendications précédentes, dans laquelle l'analogue de thrombomoduline (Solulin^{™}) présente la séquence d'acides aminés de la thrombomoduline native (SEQ ID N° : 2) modifiée aux positions suivantes :
suppression des acides aminés 1 à 3,
M388L,
R456G,
H457Q,
S474A, et
terminaison à P490.

6. Utilisation selon l'une quelconque des revendications précédentes, dans laquelle le mammifère est un être humain.


### SEQUENCE LISTING

<110> Festoff, Barry W.
   Morser, Michael J.
<120> Thrombomodulin Analogs for Use in Recovery of Spinal Cord Injury
<130> 51960AUSM1
<150> 60/229,714
   <151> 2000-08-31
<160> 2
<170> PatentIn version 3.1
<210> 1
   <211> 3466
   <212> DNA
   <213> Homo sapiens
<220>
   <221> CDS
   <222> (151)..(1875)
   <223>
<220>
   <221> mat_peptide
   <222> (205)..()
   <223>
<400> 1
   caggggctgc gcgcagcggc aagaagtgtc tgggctggga cggacaggag aggctgtcgc      60
   catcggcgtc ctgtgcccct ctgctccggc acggccctgt cgcagtgccc gcgctttccc     120
   cggcgcctgc acgcggcgcg cctgggtaac atg ctt ggg gtc ctg gtc ctt ggc      174
                                    Met Leu Gly Val Leu Val Leu Gly
                                                -15
   gcg ctg gcc ctg gcc ggc ctg ggg ttc ccc gca ccc gca gag ccg cag       222
   Ala Leu Ala Leu Ala Gly Leu Gly Phe Pro Ala Pro Ala Glu Pro Gln
   -10                 -5                  -1  1               5
   ccg ggt ggc agc cag tgc gtc gag cac gac tgc ttc gcg ctc tac ccg       270
   Pro Gly Gly Ser Gln Cys Val Glu His Asp Cys Phe Ala Leu Tyr Pro
               10                  15                  20
   ggc ccc gcg acc ttc ctc aat gcc agt cag atc tgc gac gga ctg cgg       318
   Gly Pro Ala Thr Phe Leu Asn Ala Ser Gln Ile Cys Asp Gly Leu Arg
           25                  30                  35
   ggc cac cta atg aca gtg cgc tcc tcg gtg gct gcc gat gtc att tcc       366
   Gly His Leu Met Thr Val Arg Ser Ser Val Ala Ala Asp Val Ile Ser
       40                  45                  50
   ttg cta ctg aac ggc gac ggc ggc gtt ggc cgc cgg cgc ctc tgg atc        414
   Leu Leu Leu Asn Gly Asp Gly Gly Val Gly Arg Arg Arg Leu Trp Ile
   55                  60                  65                  70
   ggc ctg cag ctg cca ccc ggc tgc ggc gac ccc aag cgc ctc ggg ccc        462
   Gly Leu Gln Leu Pro Pro Gly Cys Gly Asp Pro Lys Arg Leu Gly Pro
                   75                  80                  85
   ctg cgc ggc ttc cag tgg gtt acg gga gac aac aac acc agc tat agc        510
   Leu Arg Gly Phe Gln Trp Val Thr Gly Asp Asn Asn Thr Ser Tyr Ser
               90                  95                  100
   agg tgg gca cgg ctc gac ctc aat ggg gct ccc ctc tgc ggc ccg ttg        558
   Arg Trp Ala Arg Leu Asp Leu Asn Gly Ala Pro Leu Cys Gly Pro Leu
           105                 110                 115
   tgc gtc gct gtc tcc gct gct gag gcc act gtg ccc agc gag ccg atc        606
   Cys Val Ala Val Ser Ala Ala Glu Ala Thr Val Pro Ser Glu Pro Ile
       120                 125                 130
   tgg gag gag cag cag tgc gaa gtg aag gcc gat ggc ttc ctc tgc gag        654
   Trp Glu Glu Gln Gln Cys Glu Val Lys Ala Asp Gly Phe Leu Cys Glu
   135                 140                 145                 150
   ttc cac ttc cca gcc acc tgc agg cca ctg gct gtg gag ccc ggc gcc        702
   Phe His Phe Pro Ala Thr Cys Arg Pro Leu Ala Val Glu Pro Gly Ala
                   155                 160                 165
   gcg gct gcc gcc gtc tcg atc acc tac ggc acc ccg ttc gcg gcc cgc        750
   Ala Ala Ala Ala Val Ser Ile Thr Tyr Gly Thr Pro Phe Ala Ala Arg
               170                 175                 180
   gga gcg gac ttc cag gcg ctg ccg gtg ggc agc tcc gcc gcg gtg gct        798
   Gly Ala Asp Phe Gln Ala Leu Pro Val Gly Ser Ser Ala Ala Val Ala
           185                 190                 195
   ccc ctc ggc tta cag cta atg tgc acc gcg ccg ccc gga gcg gtc cag        846
   Pro Leu Gly Leu Gln Leu Met Cys Thr Ala Pro Pro Gly Ala Val Gln
       200                 205                 210
   ggg cac tgg gcc agg gag gcg ccg ggc gct tgg gac tgc agc gtg gag        894
   Gly His Trp Ala Arg Glu Ala Pro Gly Ala Trp Asp Cys Ser Val Glu
   215                 220                 225                 230
   aac ggc ggc tgc gag cac gcg tgc aat gcg atc cct ggg gct ccc cgc        942
   Asn Gly Gly Cys Glu His Ala Cys Asn Ala Ile Pro Gly Ala Pro Arg
                   235                 240                 245
   tgc cag tgc cca gcc ggc gcc gcc ctg cag gca gac ggg cgc tcc tgc        990
   Cys Gln Cys Pro Ala Gly Ala Ala Leu Gln Ala Asp Gly Arg Ser Cys
               250                 255                 260
   acc gca tcc gcg acg cag tcc tgc aac gac ctc tgc gag cac ttc tgc       1038
   Thr Ala Ser Ala Thr Gln Ser Cys Asn Asp Leu Cys Glu His Phe Cys
           265                 270                 275
   gtt ccc aac ccc gac cag ccg ggc tcc tac tcg tgc atg tgc gag acc       1086
   Val Pro Asn Pro Asp Gln Pro Gly Ser Tyr Ser Cys Met Cys Glu Thr
           280                 285                 290
   ggc tac cgg ctg gcg gcc gac caa cac cgg tgc gag gac gtg gat gac     1134
   Gly Tyr Arg Leu Ala Ala Asp Gln His Arg Cys Glu Asp Val Asp Asp
   295                 300                 305                 310
   tgc ata ctg gag ccc agt ccg tgt ccg cag cgc tgt gtc aac aca cag     1182
   Cys Ile Leu Glu Pro Ser Pro Cys Pro Gln Arg Cys Val Asn Thr Gln
                   315                 320                 325
   ggt ggc ttc gag tgc cac tgc tac cct aac tac gac ctg gtg gac ggc     1230
   Gly Gly Phe Glu Cys His Cys Tyr Pro Asn Tyr Asp Leu Val Asp Gly
               330                 335                 340
   gag tgt gtg gag ccc gtg gac ccg tgc ttc aga gcc aac tgc gag tac     1278
   Glu Cys Val Glu Pro Val Asp Pro Cys Phe Arg Ala Asn Cys Glu Tyr
           345                 350                 355
   cag tgc cag ccc ctg aac caa act agc tac ctc tgc gtc tgc gcc gag     1326
   Gln Cys Gln Pro Leu Asn Gln Thr Ser Tyr Leu Cys Val Cys Ala Glu
       360                 365                 370
   ggc ttc gcg ccc att ccc cac gag ccg cac agg tgc cag atg ttt tgc     1374
   Gly Phe Ala Pro Ile Pro His Glu Pro His Arg Cys Gln Met Phe Cys
   375                 380                 385                 390
   aac cag act gcc tgt cca gcc gac tgc gac ccc aac acc cag gct agc     1422
   Asn Gln Thr Ala Cys Pro Ala Asp Cys Asp Pro Asn Thr Gln Ala Ser
                   395                 400                 405
   tgt gag tgc cct gaa ggc tac atc ctg gac gac ggt ttc atc tgc acg     1470
   Cys Glu Cys Pro Glu Gly Tyr Ile Leu Asp Asp Gly Phe Ile Cys Thr
               410                 415                 420
   gac atc gac gag tgc gaa aac ggc ggc ttc tgc tcc ggg gtg tgc cac     1518
   Asp Ile Asp Glu Cys Glu Asn Gly Gly Phe Cys Ser Gly Val Cys His
           425                 430                 435
   aac ctc ccc ggt acc ttc gag tgc atc tgc ggg ccc gac tcg gcc ctt     1566
   Asn Leu Pro Gly Thr Phe Glu Cys Ile Cys Gly Pro Asp Ser Ala Leu
       440                 445                 450
   gtc cgc cac att ggc acc gac tgt gac tcc ggc aag gtg gac ggt ggc     1614
   Val Arg His Ile Gly Thr Asp Cys Asp Ser Gly Lys Val Asp Gly Gly
   455                 460                 465                 470
   gac agc ggc tct ggc gag ccc ccg ccc agc ccg acg ccc ggc tcc acc     1662
   Asp Ser Gly Ser Gly Glu Pro Pro Pro Ser Pro Thr Pro Gly Ser Thr
                   475                 480                 485
   ttg act cct ccg gcc gtg ggg ctc gtg cat tcg ggc ttg ctc ata ggc     1710
   Leu Thr Pro Pro Ala Val Gly Leu Val His Ser Gly Leu Leu Ile Gly
               490                 495                 500
   atc tcc atc gcg agc ctg tgc ctg gtg gtg gcg ctt ttg gcg ctc ctc     1758
   Ile Ser Ile Ala Ser Leu Cys Leu Val Val Ala Leu Leu Ala Leu Leu
           505                 510                 515
   tgc cac ctg cgc aag aag cag ggc gcc gcc agg gcc aag atg gag tac      1806
   Cys His Leu Arg Lys Lys Gln Gly Ala Ala Arg Ala Lys Met Glu Tyr
       520                 525                 530
   aag tgc gcg gcc cct tcc aag gag gta gtg ctg cag cac gtg cgg acc      1854
   Lys Cys Ala Ala Pro Ser Lys Glu Val Val Leu Gln His Val Arg Thr
   535                 540                 545                 550
   gag cgg acg ccg cag aga ctc tgagcggcct ccgtccagga gcctggctcc        1905
   Glu Arg Thr Pro Gln Arg Leu
                   555
   gtccaggagc ctgtgcctcc tcacccccag ctttgctacc aaagcacctt agctggcatt   1965
   acagctggag aagaccctcc ccgcaccccc caagctgttt tcttctattc catggctaac   2025
   tggcgagggg gtgattagag ggaggagaat gagcctcggc ctcttccgtg acgtcactgg   2085
   accactgggc aatgatggca attttgtaac gaagacacag actgcgattt gtcccaggtc   2145
   ctcactaccg ggcgcaggag ggtgagcgtt attggtcggc agccttctgg gcagaccttg   2205
   acctcgtggg ctagggatga ctaaaatatt tatttttttt aagtatttag gtttttgttt   2265
   gtttcctttg ttcttacctg tatgtctcca gtatccactt tgcacagctc tccggtctct   2325
   ctctctctac aaactcccac ttgtcatgtg acaggtaaac tatcttggtg aatttttttt   2385
   tcctagccct ctcacattta tgaagcaagc cccacttatt ccccattctt cctagttttc   2445
   tcctcccagg aactgggcca actcacctga gtcaccctac ctgtgcctga ccctacttct   2505
   tttgctctta gctgtctgct cagacagaac ccctacatga aacagaaaca aaaacactaa   2565
   aaataaaaat ggccatttgc tttttcacca gatttgctaa tttatcctga aatttcagat   2625
   tcccagagca aaataatttt aaacaaaggt tgagatgtaa aaggtattaa attgatgttg   2685
   ctggactgtc atagaaatta cacccaaaga ggtatttatc tttactttta aacagtgagc   2745
   ctgaattttg ttgctgtttt gatttgtact gaaaaatggt aattgttgct aatcttctta   2805
   tgcaatttcc ttttttgtta ttattactta tttttgacag tgttgaaaat gttcagaagg    2865
   ttgctctaga ttgcgagaag agacaaacac ctcccaggag acagttcaag aaagcttcaa   2925
   actgcatgat tcatgccaat tagcaattga ctgtcactgt tccttgtcac tggtagacca   2985
   aaataaaacc gactctactg gtcttgtgga attgggagct tgggaatgga tcctggagga   3045
   tgcccaatta gggcctagcc ttaatcaggt cctcagagaa tttctaccat ttcagagagg   3105
   ccttttggaa tgtggcccct gaacaagaat tggaagctgc cctgcccatg ggagctggtt   3165
   agaaatgcag aatcctaggc tccaccccat ccagttcatg agaatctata tttaacaaga   3225
   tctgcagggg gtgtgtctgc tcagtaattt gaggacaacc attccagact gcttccaatt   3285
   ttctggaata catgaaatat agatcagtta taagtagcag gccaagtcag gcccttattt   3345
   tcaagaaact gaggaatttt ctttgtgtag ctttgctctt tggtagaaaa ggctaggtac 3405
   acagctctag acactgccac acagggtctg caaggtcttt ggttcagcta agccggaatt 3465
   c 3466
<210> 2
   <211> 575
   <212> PRT
   <213> Homo sapiens
<400> 2
   Met Leu Gly Val Leu Val Leu Gly Ala Leu Ala Leu Ala Gly Leu Gly
               -15                 -10                 -5
   Phe Pro Ala Pro Ala Glu Pro Gln Pro Gly Gly Ser Gln Cys Val Glu
       -1  1               5                   10
   His Asp Cys Phe Ala Leu Tyr Pro Gly Pro Ala Thr Phe Leu Asn Ala
   15                  20                  25                  30
   Ser Gln Ile Cys Asp Gly Leu Arg Gly His Leu Met Thr Val Arg Ser
                   35                  40                  45
   Ser Val Ala Ala Asp Val Ile Ser Leu Leu Leu Asn Gly Asp Gly Gly
               50                  55                  60
   Val Gly Arg Arg Arg Leu Trp Ile Gly Leu Gln Leu Pro Pro Gly Cys
           65                  70                  75
   Gly Asp Pro Lys Arg Leu Gly Pro Leu Arg Gly Phe Gln Trp Val Thr
       80                  85                  90
   Gly Asp Asn Asn Thr Ser Tyr Ser Arg Trp Ala Arg Leu Asp Leu Asn
   95                  100                 105                 110
   Gly Ala Pro Leu Cys Gly Pro Leu Cys Val Ala Val Ser Ala Ala Glu
                   115                 120                 125
   Ala Thr Val Pro Ser Glu Pro Ile Trp Glu Glu Gln Gln Cys Glu Val
               130                 135                 140
   Lys Ala Asp Gly Phe Leu Cys Glu Phe His Phe Pro Ala Thr Cys Arg
           145                 150                 155
   Pro Leu Ala Val Glu Pro Gly Ala Ala Ala Ala Ala Val Ser Ile Thr
       160                 165                 170
   Tyr Gly Thr Pro Phe Ala Ala Arg Gly Ala Asp Phe Gln Ala Leu Pro
   175                 180                 185                 190
   Val Gly Ser Ser Ala Ala Val Ala Pro Leu Gly Leu Gln Leu Met Cys
                   195                 200                 205
   Thr Ala Pro Pro Gly Ala Val Gln Gly His Trp Ala Arg Glu Ala Pro
               210                 215                 220
   Gly Ala Trp Asp Cys Ser Val Glu Asn Gly Gly Cys Glu His Ala Cys
           225                 230                 235
   Asn Ala Ile Pro Gly Ala Pro Arg Cys Gln Cys Pro Ala Gly Ala Ala
       240                 245                 250
   Leu Gln Ala Asp Gly Arg Ser Cys Thr Ala Ser Ala Thr Gln Ser Cys
   255                 260                 265                 270
   Asn Asp Leu Cys Glu His Phe Cys Val Pro Asn Pro Asp Gln Pro Gly
                   275                 280                 285
   Ser Tyr Ser Cys Met Cys Glu Thr Gly Tyr Arg Leu Ala Ala Asp Gln
               290                 295                 300
   His Arg Cys Glu Asp Val Asp Asp Cys Ile Leu Glu Pro Ser Pro Cys
           305                 310                 315
   Pro Gln Arg Cys Val Asn Thr Gln Gly Gly Phe Glu Cys His Cys Tyr
       320                 325                 330
   Pro Asn Tyr Asp Leu Val Asp Gly Glu Cys Val Glu Pro Val Asp Pro
   335                 340                 345                 350
   Cys Phe Arg Ala Asn Cys Glu Tyr Gln Cys Gln Pro Leu Asn Gln Thr
                   355                 360                 365
   Ser Tyr Leu Cys Val Cys Ala Glu Gly Phe Ala Pro Ile Pro His Glu
               370                 375                 380
   Pro His Arg Cys Gln Met Phe Cys Asn Gln Thr Ala Cys Pro Ala Asp
           385                 390                 395
   Cys Asp Pro Asn Thr Gln Ala Ser Cys Glu Cys Pro Glu Gly Tyr Ile
       400                 405                 410
   Leu Asp Asp Gly Phe Ile Cys Thr Asp Ile Asp Glu Cys Glu Asn Gly
   415                 420                 425                 430
   Gly Phe Cys Ser Gly Val Cys His Asn Leu Pro Gly Thr Phe Glu Cys
                   435                 440                 445
   Ile Cys Gly Pro Asp Ser Ala Leu Val Arg His Ile Gly Thr Asp Cys
               450                 455                 460
   Asp Ser Gly Lys Val Asp Gly Gly Asp Ser Gly Ser Gly Glu Pro Pro
           465                 470                 475
   Pro Ser Pro Thr Pro Gly Ser Thr Leu Thr Pro Pro Ala Val Gly Leu
       480                 485                 490
   Val His Ser Gly Leu Leu Ile Gly Ile Ser Ile Ala Ser Leu Cys Leu
   495                 500                 505                 510
   Val Val Ala Leu Leu Ala Leu Leu Cys His Leu Arg Lys Lys Gln Gly
                   515                 520                 525
   Ala Ala Arg Ala Lys Met Glu Tyr Lys Cys Ala Ala Pro Ser Lys Glu
               530                 535                 540
   Val Val Leu Gln His Val Arg Thr Glu Arg Thr Pro Gln Arg Leu
           545                 550                 555